# EUROPEAN PATENT APPLICATION

(11) **EP 4 686 467 A1**
(43) Date of publication of application: **04.02.2026**
(21) Application number: 24306276.7
(22) Date of filing: 29.07.2024
(51) Int. Cl.: A61K 8/64, A61K 8/86, A61Q 5/06

(54) **A PROCESS FOR HAIR SHAPING USING SILK NANOFILM**

(71) Applicant: Ella, 75017 Paris (FR); Trustees Of Tufts College, Medford, MA 02155 (US)
(72) Inventor: Segura, Lucas, 75017 Paris (FR); Sahoo, Jugal Kishore, Medford, MA, 02155 (US); Gordon, Edward, Medford, MA, 02155 (US); Kaplan, David, Medford, MA, 02155 (US); Gopalakrishnan, Sanjana, Medford, MA, 02155 (US)
(74) Representative: Cabinet Becker et Associés

(57) **Abstract**

The present invention relates to the techniques of hair styling. In particular, the invention provides a hair treatment combining a silk fibroin hydrolysate and high molecular weight polyethylene glycols (PEGs) in a sequential process.

## Description

The present invention relates to the techniques of hair styling.

### BACKGROUND OF THE INVENTION

Consumers often wish to change the shape or the style of their hair. There are many techniques and compositions for styling or altering the shape of hair. For example, hair care products referred to as "hair relaxers" or "hair straighteners" can relax or straighten curly or kinky hair, including wavy hair. Straightening or relaxing the curls of very curly hair may increase the manageability and ease of styling of such hair. Compositions for permanent waving the hair will impart a curl or a wave to otherwise straight hair. Different types of compositions can be applied onto hair in order to change its shape and make it more manageable, such as alkaline and acidic compositions. Hair relaxers, straighteners, perms, and/or waves may either be applied in a hair salon by a professional or in the home by the individual consumer.

Most hair straightening and curling treatment, more specifically, permanent and semipermanent hair straightening treatment current treatments, act upon the disulfide bond network of the keratin or reticulating keratin (so-called Brazilian treatment), with small chemical molecules that may be toxic and may damage the hair. The need is to achieve similar outcomes but with safer and greener methods to match consumer needs along with effective hair treatments.

Alternative treatments are thus needed.

### SUMMARY OF THE INVENTION

The inventors provide a hair treatment based on the spontaneous formation of a silk-nanofilm on the surface of keratin fibers, optionally after disulfide bond network reduction.

More particularly, the inventors propose to combine a silk fibroin hydrolysate and high molecular weight polyethylene glycols (PEGs) in a sequential process.

A subject of the invention is a process for shaping the hair comprising:
i) applying a composition A comprising a silk fibroin hydrolysate, followed by
ii) applying a composition B comprising one or more polyethylene glycols (PEGs) having a molecular weight greater than or equal to 10 kDa.

Composition B is applied after composition A, in a sequential manner. Composition A and composition B may have different leave-on times.

In a particular embodiment, composition A may be rinsed off before composition B is applied. In another embodiment, composition B is applied without rinsing composition A.

The process of the invention may further comprise the application of a pre-treatment composition to the hair prior to step i), i.e. prior to application of composition A. This step is advantageously designed to open hair cuticle.

In a particular embodiment, the process is a process for curling the hair.

In another embodiment, the process is a process for straightening the hair.

In yet another embodiment, the process is a process for styling the hair.

According to the inventors, using the PEG allows to enhance the crystallinity of the film formed by silk fibroin hydrolysate on the keratin fibers. Such greater crystallinity offers a better barrier to humidity and thus improves how shapable the hair can be, and how the shaping can be maintained.

Further the process according to the invention allows to obtain a lasting effect while preserving the keratin fibers. The hair is not damaged. The keratin fibers are well individualized.

Using the process of the invention; the hair is easier to shape and/or style. The process of the invention makes it possible to retain the hair style or shape even when exposed to humid atmosphere. In particular, the process of the invention allows to reduce frizziness and to obtain better curl definition.

In particular, the shaping achieved with the process of the invention is remanent to shampoos, i.e. the hair come back to their styled shape upon drying after a shampoo (i.e. after natural drying and/or blow drying).

The process of the invention also provides good conditioning properties to the keratin fibers, such as shine, smoothness and soft feel, as well as a natural appearance.

### BRIEF DESCRIPTION OF THE DRAWINGS

**Figure 1****:** Hair treated with silk fibroin hydrolysate solution (without post-treatment with solution comprising high molecular weight PEG) and straightened with a flat iron: photo taken before exposure to high humidity.
**Figure 2****:** Hair treated with silk fibroin hydrolysate solution (without post-treatment with solution comprising high molecular weight PEG) and straightened with a flat iron: photo taken after exposure to high humidity.
**Figure 3****:** Hair treated with silk fibroin hydrolysate solution followed by post-treatment with a solution comprising PEG 10KDa and straightened with a flat iron: photo taken before exposure to high humidity.
**Figure 4****:** Hair treated with silk fibroin hydrolysate solution followed by post-treatment with a solution comprising PEG 10KDa and straightened with a flat iron: photo taken after exposure to high humidity.
**Figure 5A****:** Attenuated Total Reflection Fourier Transform Infra-red (ATR-FTIR) measurements of films prepared with solution comprising silk fibroin hydrolysate (30 mb (2 wt%)) followed by post-treatment with solutions comprising different polyethylene glycol (PEG) chain length (2000, 10000, 20000, 200000; 4 wt%).
**Figure 5B****:** Strong absorption peak at β-sheet region (1616-1637 cm-1) was observed with increase in PEG chain length. Percentage β-sheet content in different composite films with different PEG molecular weight. % β-sheet content increased with increase in PEG molecular weight. Data are presented as mean ± standard deviation (n = 3).

### DETAILED DESCRIPTION OF THE INVENTION

The process of the invention makes use of at least two compositions:
- a composition A comprising a silk fibroin hydrolysate, and
- a composition B comprising one or more polyethylene glycols (PEGs) having a molecular weight greater than or equal to 10 kDa.

These compositions A and B are described in greater detail below.

### Composition A

The process according to the invention comprises i) applying to the hair a composition A comprising a silk fibroin hydrolysate.

The organism producing the silk may be a worm, a spider, or any organism that has been genetically modified to produce silk.

In a preferred embodiment, the silk is from a silkworm, preferably *Bombyx mori,* while other silkworm species can also be used such as *Antheraea mylitta, Antheraea assamensis, Antheraea pernyi, Samia cynthia, Philosamia ricini, Antheraea yamamai, Antheraea paphia.* In another embodiment, the silk is from a spider, such as *Caerostris darwini, Nephila clavipes, Araneus diadematus, Latrodectus mactans, Argiope argentata.*

Methods for preparing silk fibroin hydrolysates are well known in the art. For instance, the silk fibroin hydrolysate may be obtained as described in Rockwood et al. (Materials fabrication from Bombyx mori silk fibroin. Nat Protoc. 2011 Sep 22;6(10): 1612-31). Briefly, the process involves degumming the silk, for instance by boiling, from the silk sericin matrix when present, and solubilization into a chaotropic solvent, for instance in a lithium bromide solution. The hydrolysate can then be made metastable by partially or entirely removing the chaotropic elements through methods such as dialysis or extraction.

Methods for obtaining silk fibroin hydrolysates are also described in Wray et al, 2011. Effect of processing on silk-based biomaterials: Reproducibility and biocompatibility. J Biomed Mater Res Part B 2011:99B:89-101. In Wray et al, 2011, five grams of B. mori silkworm cocoons were immersed into 1 L of boiling 0.02 M Na₂CO₃ solution for 5, 30, or 60 min. These samples will be hereafter referred to as 5 mb, 30 mb, and 60 mb, where "mb" stands for "minutes-boiled."

Similarly, in a particular embodiment, the silk fibroin hydrolysate useful in the present invention may have a boiling time (mb) value that ranges from 10mB to 460mB, preferably from 30mB to 120mB.

Composition A may comprise additional ingredients, such as lithium salts (e.g. lithium bromide, lithium chloride), calcium salts (e.g. calcium chloride, calcium nitrate), potassium salts, 1-butyl-3-methylimidazolium chloride, 1-butyl-2,3-dimethylimidazolium, and 1-ethyl-3-methylimidazolium, hexafluoroisopropanol and hexafluoroacetone hydrate, acids such as acetic acid, formic acid, or trifluoroacetic acid, surfactants such as cationic, anionic, zwitterionic or non-ionic surfactants, as well as saccharides, alcohol, urea, and mixtures thereof.

For instance, composition A may comprise lithium salts, in particular lithium bromide.

In a particular embodiment, composition A may comprise cationic surfactants, in particular ethyl lauryl arginine hydrochloride, preferably in a content ranging from 1% to 50% by weight, relative to total weight of said composition A.

In another embodiment, composition A may comprise non-ionic surfactants, in particular Triton X-100.

In another particular embodiment, composition A may comprise alcohol, in particular alkyl monohydric alcohols such as butanol, preferably in a content ranging from 1% to 50% by weight, relative to total weight of said composition A.

In another particular embodiment, composition A may comprise urea, preferably in a content ranging from 0.1% to 10% by weight, relative to total weight of said composition A.

In another particular embodiment, composition A may comprise PEGs having a molecular weight higher than 200Da and strictly lower than 10kDa.

In a particular embodiment, silk fibroin hydrolysate is present in a total content that ranges from 0.1% to 15%, preferably from 2% to 10% by weight, relative to total weight of said composition A.

### Composition B

The process according to the invention comprises ii) applying to the hair a composition B comprising one or more polyethylene glycols (PEGs) having a molecular weight greater than or equal to 10 kDa.

In a particular embodiment, the PEGs having a molecular weight greater than or equal to 10 kDa preferably have a molecular weight of between 10 and 100 kDa, preferably between 10 and 50kDa, more preferably between 10 and 20 kDa.

In a particular embodiment, the PEGs having a molecular weight greater than or equal to 10 kDa are present in a total content that ranges from 0.1 % to 100% by weight, preferably from 0.5 to 50% by weight, more preferably from 2% to 10% by weight relative to total weight of said composition B.

Composition B may comprise additional ingredients, such as cationic, anionic, zwitterionic and non-ionic surfactants, alcohol, urea, and mixtures thereof.

Composition B may further comprise naturally occurring oligosaccharides such as sucrose, lactose, maltose, raffinose, stachyose, and mixtures thereof.

Composition B may also further comprise polysaccharides like starch, cellulose, glycogen, chitin, inulin, pectin, hyaluronic acid, alginates, and mixtures thereof.

### Pre-treatment composition

The process according to the invention may further comprise applying a pre-treatment composition to the hair prior to step i) (i.e. prior to application of composition A).

Preferably, this pre-treatment composition may comprise surfactants, and/or urea.

Preferably, the pre-treatment composition may further comprise crosslinking or reducing agents such as thioglycolic acid, thioglycolates, cysteine salts or free bases, mercaptans, glutathione, aldehydes, difunctional Michael acceptors, and mixture thereof, preferably in a content ranging from 0.1 to 20%, more preferably from 5 to 15% by weight, relative to total weight of said pre-treatment composition.

Preferably, the pre-treatment composition has a pH ranging from 7.5 to 11.

### Process

The process of the invention is a process for shaping or styling the hair, comprising:
i) applying a composition A comprising a silk fibroin hydrolysate, followed by
ii) applying a composition B comprising one or more PEGs having a molecular weight greater than or equal to 10 kDa.

Therefore, the process of the invention involves at least two steps i) and ii), wherein compositions A and B are applied sequentially onto the hair.

In a preferred aspect, composition A is applied according to step i) for a leave-on time of between 1 and 90 minutes, preferably between 1 and 60 minutes, more preferably between 1 and 45 minutes, even more preferably between 1 and 30 minutes.

Preferably, composition A is applied onto wet hair.

In a preferred aspect, composition B is applied according to step ii) for a leave-on time of between 1 and 90 minutes, preferably between 1 and 60 minutes, more preferably between 1 and 45 minutes, even more preferably between 1 and 30 minutes.

In a particular aspect of the invention, step i) is immediately followed by step ii). In other words, the application of composition A comprising a silk fibroin hydrolysate is immediately followed by the application of composition B comprising one or more polyethylene glycols (PEGs) having a molecular weight greater than or equal to 10 kDa, i.e. there is no rinsing step between step i) and step ii).

In another particular aspect of the invention, the process may further comprise a rinsing step between step i) and ii). In other words, in this aspect, the process of the invention comprises:
- applying a composition A comprising a silk fibroin hydrolysate,
- rinsing, preferably with water, and
- applying a composition B comprising one or more PEGs having a molecular weight greater than or equal to 10 kDa.

Preferably, step ii), i.e. the application of composition B, may be followed by a rinsing step, preferably with water. Then the hair may be dried, either naturally or typically with a blow drier. In a particular embodiment, the process of the invention further comprises the application of a pre-treatment composition to the hair prior to step i) (i.e. prior to application of composition A). Such pre-treatment composition is described above.

The process of the invention is a hair shaping process. In other words, the aim of the invention is to shape or style the hair after the process of the invention has been carried out.

Shaping and styling the hair may be carried out by any conventional means.

In a particular aspect, the hair is curled. In another embodiment, the hair is straightened. In yet another embodiment, the hair is styled.

The invention will now be illustrated using the examples below. These examples are presented by way of indication and do not in any way limit the invention.

### EXAMPLES

### Example 1 : Silk fibroin hydrolysate preparation:

For the preparation of silk fibroin hydrolysate, cocoons of silkworm Bombyx mori were used. The preparation process involved the removal of sericin (e.g. degumming), which was performed as follow.

The cocoons were cut into dime-sized pieces using titanium scissors, and silkworms were disposed of. A total of 5 g of cocoon pieces were measured.

2L of a solution comprising 0.02M of Na₂CO₃ in water was prepared and heated until boiling. The 5 g of cut cocoon pieces were then added and boiled for 30 minutes with occasional stirring using a spatula to promote good dispersion of fibroin. The boiling time was monitored closely to ensure reproducibility and avoid silk fibroin degradation due to too long boiling time.

The silk fibroin degummed fibers were removed using a spatula and cooled by rinsing in ultrapure cold water, with excess water squeezed out of the silk.

The fibroin was then rinsed three times in 1 liter of ultrapure water for 20 minutes while gently stirring on a stir plate. This step was repeated twice for a total of three rinses. After the third rinse, the silk was squeezed well and spread out on a clean piece of aluminum foil to dry in a fume hood overnight.

Upon used, the silk fibroin was dissolved in a solution comprising 9.3M of lithium bromide (LiBr), dried degummed silk fibroin to LiBr of 1:4. The dissolution was performed in an oven at 60°C for 4 hours. The solution obtained was amber in color, transparent, and highly viscous. The solution obtained then underwent a dialysis for at least 48h and 6 water changes followed by centrifugation at 9000 rpm for 20 minutes at 4° C to remove impurities. The supernatants after centrifugation were filtered using 40 µm cell-strainer. The concentration of the resulting silk solution was measured and stored at 4° C until further use.

For dialysis and centrifugation, dialysis cassettes were hydrated in water for a few minutes, and 12 ml of the silk-LiBr solution was inserted into a 3-12 ml dialysis cassette using a 20-ml syringe and an 18-gauge needle, taking care not to puncture or touch the dialysis membrane. The solution was kept warm to ease this step and to avoid shearing that could induce β-sheet formation within the silk fibroin hydrolysate. An additional needle was used to allow air to escape during this process. The solution was dialyzed against 1 liter of ultrapure water per 12 ml cassette, using a large stir bar on a magnetic stir plate for mixing. The water was changed 6 times within 48 hours. The silk was removed from the cassettes with another 20-ml syringe and an 18-gauge needle and placed in a 50-ml conical tube.

Centrifugation was performed to remove impurities by spinning at 9,000 r.p.m. at 4°C for 20 minutes. The tubes were carefully removed from the centrifuge, and the silk solution was transferred into another centrifuge tube, ensuring any impurities, such as white flocculent or brown residues, were left behind. This step was repeated twice. To determine the concentration of the silk in solution, 0.5 ml of the silk solution was added and dried at 60°C overnight. The weight of the dried silk was divided by 0.5 ml to yield the weight per volume percentage. Typically, 5 g of silk cocoons yielded 25 ml of 7-8% (wt/vol) silk solution, which was clear with a yellow tint and slightly viscous. Impurities such as white flocculents or dark particulates were removed by recentrifugation if necessary.

### Example 2: Process using silk fibroin hydrolysate without high molecular weight PEG as post-treatment

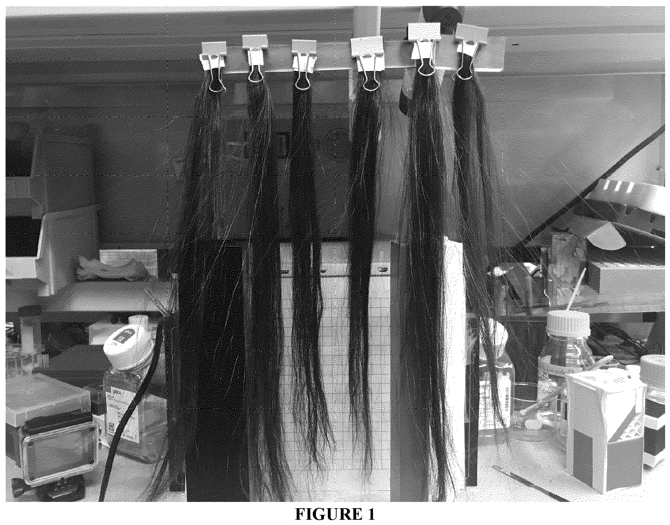

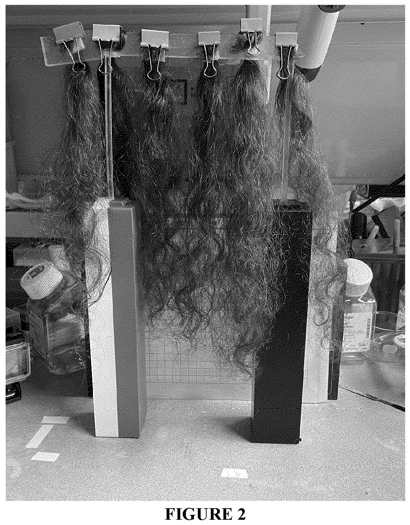

A 5g strand of natural curly hair, measuring 25 cm in length from root to tip, with the root tied together tightly, was used in this experiment. A solution of soap (2 wt%) was prepared, adjusting the pH to 8-9 using ammonium hydroxide. The soap solution (50 ml for 3-6 g of hair) was applied to the hair, massaged in, and the hair was submerged in it for 10 minutes. The hair was then thoroughly rinsed with water. A silk fibroin hydrolysate solution as prepared in Example 1 was applied at a rate of 1mL/g of hair, massaged into the hair, and left to rest for 1 hour. The hair was then thoroughly rinsed with water and blow-dried until completely dry, ensuring no dampness remained at the roots where the strands were held together to avoid compromising the test. The hair was straightened using a flat iron (**Figure 1**) and then exposed to 80% relative humidity at 25°C for 30 minutes (**Figure 2**).

After exposure to humidity, the hair significantly curled back, showing a frizzy and undisciplined aspect.

### Example 3: Process using silk fibroin hydrolysate and post-treatment with high molecular weight PEG

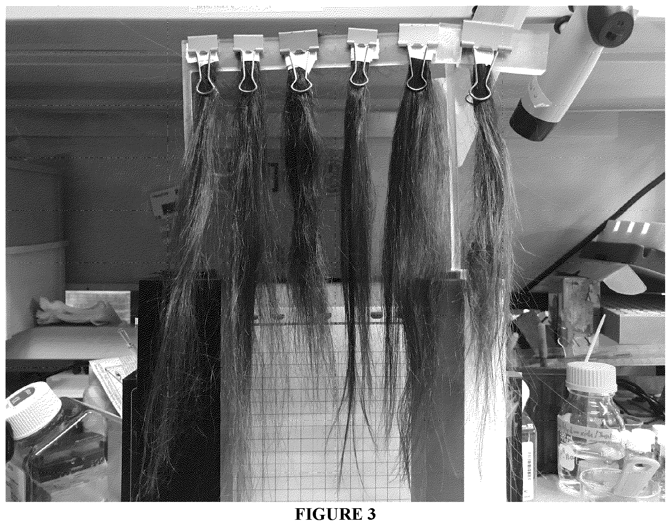

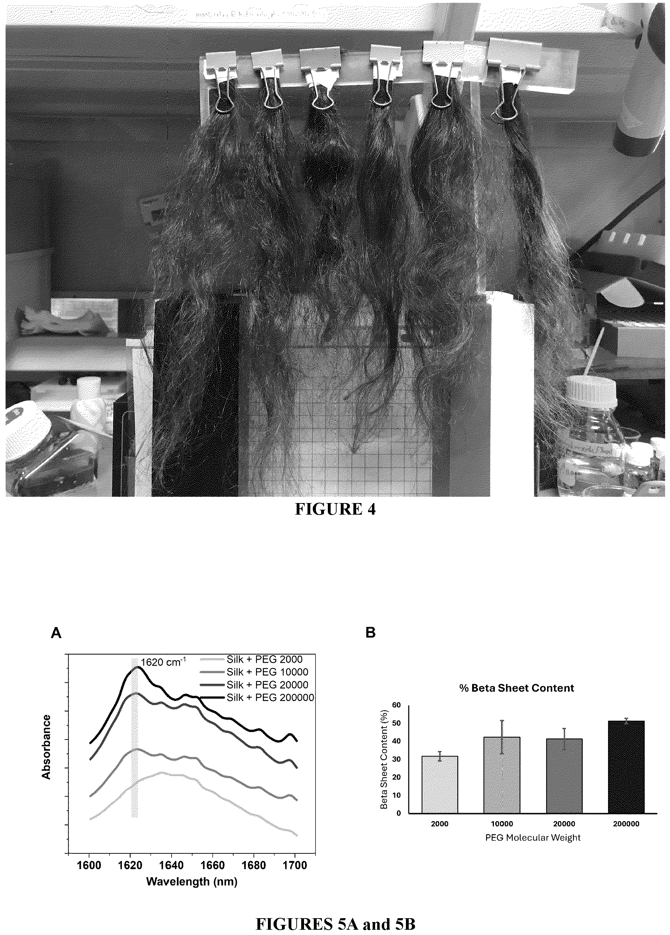

A 5g strand of natural, curly hair, measuring 25 cm in length from root to tip, with the root tied together tightly, was used in this experiment. A solution of soap (2 wt%) was prepared and adjusted the pH to 9 using ammonium hydroxide. The soap solution (50 ml for 3-6 g of hair) was applied to the hair, massaged in, and the hair was submerged in it for 10 minutes. The hair was then thoroughly rinsed. A silk fibroin hydrolysate solution as prepared in Example 1 was applied at a rate of 1mL/g of hair, massaged into the hair, and left to rest for 30 minutes. The hair was then thoroughly rinsed with water. Then, the hair strands were treated with a solution comprising 4wt% of PEG 10 kDa at a rate of 5mL per hair strand of 5grams. After 30 minutes, the hair was rinsed with water and the excess water was blotted with a paper towel. The hair was then blow-dried until completely dry, ensuring no dampness remained at the roots where the strands were held together to avoid compromising the test. The hair was straightened using a flat iron (**Figure 3**) and then exposed to 80% relative humidity at 25°C for 30 minutes (**Figure 4**).

After exposure to humidity, the hair shows significantly improved hair discipline and reduced curly and frizzy effect compared to the hair not treated with high molecular weight PEG.

### Example 4: Silk nanofilm crystallinity

### Material and Methods

### Film preparation:

The composite films were prepared by drop-casting the solution in a poly dimethyl siloxane (PDMS) mold in a fume hood. Typically, 300 µl of 2 wt% silk solution was drop-casted into the PDMS molds and were allowed to dry for 4h at room temperature. After 4h, 300 µl of freshly prepared 4 wt% aqueous PEG solutions of different molecular weights (2kDa, 10kDa, 20kDa, and 200kDa) were added to the different films and allowed to dry overnight before any further analysis.

### Fourier Transform Infra-Red Spectroscopy (FT-IR):

Protein secondary structures of as prepared films were determined using a JASCO FTIR 6200 spectrometer (JASCO, Tokyo, Japan) with a MIRacle attenuated total reflectance (ATR) with germanium crystal. FT-IR measurements were performed by averaging 128 scans with a resolution of 4 cm⁻¹ within 600-4000 cm⁻¹ wavenumber range. FTIR was performed on at least three samples (n=3) for each type of composite film. Data analysis were performed and calculated using the Fourier self-deconvolution method using Origin software (Origin 2020, OriginLab, Northampton, MA). Percentage β-sheet content was computed using methods reported previously (Ling et al,ACS Biomater. Sci. Eng. 2, 1298-1308 (2016); Guo et al, Nat. Mater. 19, 102-108 (2020)). Specifically, baseline noise was removed, four areas of protein structure (beta sheet, random coil, alpha helix, and beta turn) were assigned in the spectra and a Gaussian model was used to compute the relative level of each structure type.

### Statistics:

ATR-FTIR studies were performed on n = 3 independent sample replicates for each composite film. All data are expressed as means ± standard deviations and used to generate graphical figures. Unpaired t-test were performed using GraphPad prism (GraphPad Software, San Diego, CA) to determine statistical significance (p < 0.05).

### Results

Beta Sheet content generally increased with increasing PEG molecular weight

| PEG Molecular Weight | Beta Sheet Content |
|---|---|
| 2kDa | 31.8 ± 2.6% |
| 10kDa | 42.3 ± 9.3% |
| 20kDa | 41.3 ± 5.8% |
| 200kDa | 51.3 ± 1.5% |

A statistically significant increase in beta sheet content was found when comparing PEG 2k with PEG 200k (p=0.0004) and PEG 20k with PEG 200k (p=0.0445).

Beta sheet content ranged from ~32% to 51%.

Logarithmic line of fit displayed a high correlation for the data (R²=0.9451).

As shown on **Figure 5**, crystallinity of the silk film is directly dependent on the molecular weight of the PEG used. As the molecular weight increases, not only does the crystallinity increase, but the variance of the results also tends to diminish. Therefore, the nanofilm formed by the combination of silk with PEG of high molecular weight brings superior proprieties on the hair, in terms of shaping and resistance to humidity.

## Claims

1. A process for shaping the hair comprising:
i) applying a composition A comprising a silk fibroin hydrolysate, followed by
ii) applying a composition B comprising one or more polyethylene glycols (PEGs) having a molecular weight greater than or equal to 10 kDa.

2. The process of claim 1, wherein the silk fibroin hydrolysate is obtained from Bombyx mori or a spider.

3. The process of claim 1 or 2, wherein the silk fibroin hydrolysate has a boiling time (mb: minutes boiled) value that ranges from 10mB to 460mB, preferably from 30 mB to 120mB.

4. The process of any one of the preceding claims, wherein the silk fibroin hydrolysate is present in a total content that ranges from 0.1% to 15%, preferably from 2% to 10% by weight, relative to total weight of said composition A.

5. The process of any one of the preceding claims, wherein the PEGs comprised in composition B have a molecular weight between 10 and 100 kDa, preferably between 10 and 50kDa, more preferably between 10 and 20 kDa.

6. The process of any one of the preceding claims, wherein the PEGs are present in a total content that ranges from 0.1 % to 100% by weight, preferably from 0.5 to 50% by weight, more preferably from 2% to 10% by weight relative to total weight of said composition B.

7. The process of any one of the preceding claims, wherein said composition A is applied according to step i) for a leave-on time of between 1 and 90 minutes, preferably between 1 and 60 minutes, more preferably between 1 and 45 minutes, even more preferably between 1 and 30 minutes.

8. The process of any one of the preceding claims, wherein said composition B is applied according to step ii) for a leave-on time of between 1 and 90 minutes, preferably between 1 and 60 minutes, more preferably between 1 and 45 minutes, even more preferably between 1 and 30 minutes.

9. The process of any of claims 1 to 8, wherein the process is a process for curling the hair.

10. The process of any of claims 1 to 8, wherein the process is a process for straightening the hair.

11. The process of any of claims 1 to 8, wherein the process is a process for styling the hair.
